# EUROPÄISCHE PATENTSCHRIFT

(11) **EP 0 795 306 B1**
(45) Veröffentlichungstag und Bekanntmachung des Hinweises auf die Patenterteilung: **05.09.2001**
(21) Anmeldenummer: 96103923.7
(22) Anmeldetag: 13.03.1996
(51) Int. Cl.: A61F 2/34

(54) **Modulares Knochenimplantat mit Pfanne und Stiften**
Modular bone prosthesis with cup and pins
Prothèse osseuse modulaire comportant une cupule et des pointes

(43) Veröffentlichungstag der Anmeldung: 17.09.1997
(73) Patentinhaber: ZAHEDI, Amir G., Dr., 48301 Nottuln (DE)
(72) Erfinder: ZAHEDI, Amir G., Dr., 48301 Nottuln (DE)
(74) Vertreter: von Samson-Himmelstjerna, Friedrich R., Dipl.-Phys.

(56) Entgegenhaltungen:
- EP-A- 0 013 863
- WO-A-94/05234
- FR-A- 2 519 545
- FR-A- 2 598 908
- GB-A- 2 246 957

## Beschreibung

Die Erfindung betrifft ein Knochenimplantat sowie ein entsprechendes Verfahren jeweils nach dem Oberbegriff der Patentansprüche 1 und 5.

Ein derartiges Knochenimplantat ist beispielsweise aus der FR-2 519 545 bekannt.

Hüftimplantate in Form sogenannter "Preß-Fit-Pfannen" sind zudem beispielsweise aus der DE 42 05 018 C1 bekannt. Der exakte Sitz der Pfanne in dem Knochen des Patienten wird durch eine maßgenaue Anpassung des Knochens an die aufzunehmende Pfanne erreicht.

Die Erfindung betrifft weiterhin ein Verfahren nach dem Oberbegriff des Patentanspruches 5. Hüftpfannen mit Verankerungsstiften werden nach einem derartigen Verfahren hergestellt.

In der Praxis besteht das Problem, die Hüftpfanne möglichst genau an den Beckenknochen des Patienten anzupassen. Dieses Problem schließt die Verankerung der Hüftpfanne im Beckenknochen des Patienten ein. Aus der FR-2 519 545 ist ein Knochenimplantat bekannt, bei dem die Stifte herstellerseitig an der Pfanne angebracht sind, z. B. durch eine Preßpassung, bei der die Stifte in entsprechend genau dimensionierte Bohrungen der Pfanne eingeschlagen werden. Alternativ ist in der FR-2 519 545 vorgesehen, die Stifte mit zusätzlichen Hilfsmitteln, z. B. Schrauben, Klebstoff oder durch Verschweißen, in der Pfanne festzulegen.

Die derart herstellerseitig mit Stiften bestückte Pfanne wird während der Operation vom Arzt in den Beckenknochen des Patienten eingesetzt, wobei zunächst mittels einer geeigneten Bohrschablone Bohrungen in den Beckenknochen des Patienten eingebracht worden sind, welche später die Stifte des Knochenimplantates aufnehmen sollen. Dabei stellt sich individuell für jeden Patienten verschieden ein Aufbau des Beckenknochens heraus, der an unterschiedlichen Stellen eine unterschiedliche Dicke aufweist, so daß die Bohrungen unterschiedlich tief gestaltet werden könnten.

Gemäß der FR-2 519 545 sind jedoch die Stifte an der Hüftpfanne stets so zu bemessen, daß sie nahezu gleichzeitig in die Bohrungen eingeführt werden. Sie sind also aufgrund der kugelsegmentförmigen Pfanne und der entsprechend geformten Ausnehmung im Beckenknochen in der Regel gleich lang, so daß unterschiedlich gut tragende Eigenschaften des Beckenknochens (aufgrund seiner Dicke oder aufgrund seiner Knochenstruktur) nicht berücksichtigt werden können.

Aus der WO-A-94/05234 ist ferner ein aus der Pfanne und den Stiften bestehender Bausatz bekannt. Die Stifte werden hierbei patientspezifisch in eine Auswahl vorgefertigter Löcher gesteckt, und zwar von der Innenseite der Pfanne her und dort z.B. durch Einschrauben jeweils einer Schraube in die Stifte aufgeweitet, so daß ein Preßsitz zwischen Stift und Pfanne realisiert wird. Nachteilig ist hieran, daß die Stifte durch entsprechende Belastung von außen weiter nach innen rutschen können und durch eine zusätzlich in der Pfanne aufgenommene Schale daran gehindert werden müssen, bis zur Pfannenkugel vordringen zu können und dort einen unerwünschten Abrieb zu erzeugen. Ein ähnlicher Bausatz ist in der GB 2 246 957 A gezeigt, bei dem jedoch die innere Schale fehlt und die Stifte somit nachteilig an der Pfannenkugel reiben können.

Die in der FR-2 519 545 erwähnten Verbindungen wie Verpressen, die Verwendung zusätzlicher Schrauben, das Kleben oder Schweißen kommen für die Bausätze der WO-A-94/05234 und der GB 2 246 957 A nicht in Frage, da sie für den operierenden Arzt bei dem herrschenden Zeitdruck und unter den geforderten sterilen Operationsbedingungen in der Praxis nicht durchführbar sind.

Der Erfindung liegt die Aufgabe zugrunde, ein gattungsgemäßes Knochenimplantat und ein gattungsgemäßes Verfahren dahingehend zu verbessern, daß es optimal und schnell (d.h. ohne daß die Operationszeit wesentlich verlängert wird) an die individuelle Situation des Patienten angepaßt werden kann.

Diese der Erfindung zugrundeliegende Aufgabe wird durch ein Knochenimplantat mit den Merkmalen des Anspruches 1 und durch ein Verfahren mit den Verfahrensschritten gemäß Anspruch 5 gelöst.

Die Erfindung schlägt mit anderen Worten vor, die Hüftpfanne nicht herstellerseitig zu komplettieren, sondern als Bausatz auszugestalten und dabei die Verbindung zwischen Stiften und Pfanne durch einen Konussitz zu schaffen. Der operierende Arzt kann in Abhängigkeit von der individuellen Situation des Patienten die Pfanne mit Stiften bestücken und einsetzen, so daß je nach lokaler Stärke des Beckenknochens die optimale Bestückung der Pfanne mit Stiften erfolgen kann. Dabei kann die Lage und ggf. auch die Länge der Stifte individueller Anpassung an jeden Patienten gewählt werden, was bei einer fertig angelieferten und mit Stiften bestückten Pfanne nicht möglich ist.

Gemäß dem kennzeichnenden Verfahrensmerkmal werden die Stifte lediglich in die Pfanne eingesteckt und durch dieses Einstecken in der Pfanne festgelegt. Die bereits oben erwähnte Konusverbindung, die eine derartige Befestigung der Stifte in der Pfanne ermöglicht, ermöglicht auch bei Pfannen mit sehr kleinem Durchmesser eine werkzeuglose Befestigung der Stifte, wobei der Arzt nach dem Einsetzen des ersten Stiftes bei der Befestigung der weiteren Stifte auch bei beengten räumlichen Verhältnissen nicht durch bereits vorhandene Stifte behindert wird, wie dies beispielsweise bei einer Verschraubung der Fall wäre.

Zudem wird durch das Einstecken eine sehr schnelle Befestigungsmöglichkeit für die Stifte geschaffen, so daß die vorteilhafte individuelle Anpassung des Knochenimplantates an den jeweiligen Patienten ohne eine wesentliche Verlängerung des Operationsverlaufes erfolgen kann.

Schließlich ist es vorteilhaft, daß die Stifte in der Richtung angeordnet sind, die der Einsetzrichtung des Knochenimplantates entspricht. Auf diese Weise ist es möglich, in der bechriebenen Weise zunächst die Pfanne mit den Stiften zu bestücken und dann in den Beckenknochen des Patienten einzusetzen. Würde die Längsrichtung der Stifte von der Einsetzrichtung der Pfanne abweichen, müßte zunächst die Pfanne eingesetzt werden und anschließend von Innen die Stifte durch die Pfanne in den Knochen des Patienten eingeführt werden, wobei beispielsweise federelastische Rastmittel erforderlich wären, um die Anschläge zu bilden, die eine ins Innere der Pfanne gerichtete Bewegung der Stifte abstützen und auffangen würden, oder wobei die in der WO-A-94/05234 offenbarte innere Kugelschale erforderlich wäre.

Durch den Konussitz wird auch auf kleinstem Raum, z.B. bei Pfannen mit nicht mehr als 44 mm Durchmesser, ein sicheres und rasches Einsetzen der Stifte in die Pfanne ermöglicht. Die Konusverbindung stellt zudem eine feste Verbindung zwischen Stift und Pfanne dar, wobei durch das Einführen der Pfanne in den Beckenknochen des Patienten diese Verbindung druckbelastet wird und dadurch während der Implantation der Pfanne noch verbessert wird. Die im anschließenden Gebrauch auftretende Belastung der Pfanne stellt keine Zug- sondern eine Druckbelastung dar, so daß die Konusverbindung für die in der Praxis auftretenden Belastungen des Knochenimplantates eine stabile und allen Anforderungen gerecht werdende Verbindung zwischen Stiften und Pfanne darstellt.

Verschraubte Befestigungsmittel können schon während ihrer Anbringung zu Abrieb durch die Schraubbewegung führen. Dieser Antrieb führt einerseits zu einem vorzeitigen Verschleiß und kann andererseits Entzündungen hervorrufen, so daß eine erneute Operation zu einem unerwünscht frühen Zeitpunkt erforderlich wird. Der erhebliche Durchmesserunterschied zwischen dem mit Gewinde versehenen Bereich und dem Schaft des Befestigungsmittels bewirkt eine Spannungskonzentration am Übergang dieser beiden unterschiedlichen Durchmesser, so daß je nach auftretenden Belastungen eine Beschädigung in diesem Bereich auftreten kann, so daß die Befestigungsmittel zerstört werden können, wodurch wiederum eine Lockerung des Implantats sowie Abrieb hervorgerufen werden kann, so daß auch hierdurch unerwünscht frühe Nachoperationen erforderlich werden können.

Vorteilhaft weisen die Stifte unterschiedliche Längen auf. Dadurch ist in Übereinstimmung mit dem vorgeschlageenen Verfahren möglich, die Stifte individuell an die Situation des jeweiligen Patienten anzupassen, indem unterschiedliche Bohrlochlängen im Beckenknochen ermittelt und durch die jeweils ausgewählte Stiftlänge optimal ausgenutzt werden können. Auf diese Weise wird ein maximaler Halt der Hüftpfanne im Beckenknochen ermöglicht.

In der FR-2 519 545 ist ein Kreissegment von Stiften und von die Stifte aufnehmenden Ausnehmungen ausgespart. Jede in der Pfanne vorgesehene Ausnehmung ist auch mit einem Stift bestückt. Im Gegensatz zu dieser Lehre schlägt die Erfindung vorteilhaft vor, die Anzahl der Ausnehmungen größer vorzusehen als die Anzahl der an der Pfanne befestigten Stifte. Es kann also die Pfanne mit einer relativ großen Anzahl von Ausnehmungen versehen sein, um an den für den jeweiligen Patienten optimalen Stellen Stifte im Beckenknochen anordnen zu können. Die individuell durch den Arzt vorgenommene Bestückung der Pfanne mit den Stiften ermöglicht dabei, diese optimalen Einsatzpunkte für die Stifte zu verwenden.

Ausführungsbeispiele der Erfindung werden anhand der Zeichnungen im folgenden näher erläutert. Dabei zeigt
- Fig. 1: eine Draufsicht auf eine Pfanne und
- Fig. 2: eine Seitenansicht auf eine Pfanne ähnlich der von Fig. 1, wobei einige Bereiche der Pfanne weggebrochen dargestellt sind.

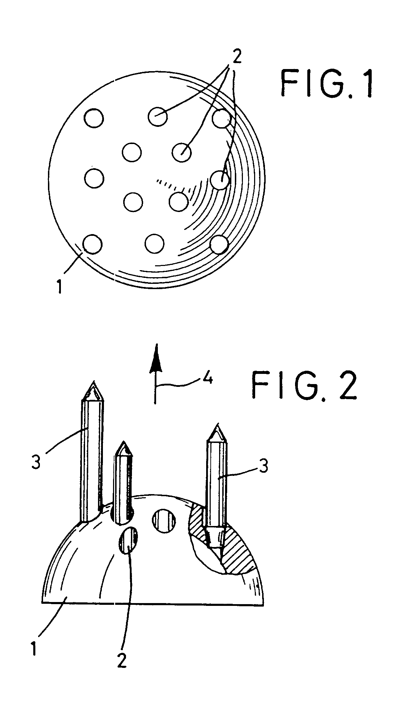

In Fig. 1 ist mit 1 eine Pfanne bezeichnet, die in einen Beckenknochen eines Patienten eingesetzt werden kann und welche als sogenannte "Press-Fit-Pfanne" ausgebildet ist. Die Pfanne 1 weist mehrere achsparallele und konisch verlaufende Bohrungen 2 auf.

Wie aus Fig. 2 ersichtlich ist, sind die Befestigungsmittel als Stifte 3 ausgebildet. Die Stifte 3 sind insgesamt gewindelos ausgebildet und weisen einen konischen unteren Bereich auf, mit dem sie in die Bohrungen 2 der Pfanne 1 eingesetzt werden können.

In Anpassung an die jeweils bei jedem einzelnen Patienten vorliegenden Verhältnisse sind verschieden lange Stifte 3 ausgewählt, so daß die Bestückung der Bohrungen 2 mit den verschiedensten Stiften 3 in Abhängigkeit von den jeweils beim Patienten vorherrschenden Verhätnissen erfolgen kann.

Mit 4 ist in Fig. 2 die Einsetzrichtung angedeutet, in welcher die Pfanne 1 in den Beckenknochen des Patienten eingesetzt wird. Dabei ist ersichtlich, daß die Achsrichtung sowohl der Stifte 3 als auch der Bohrungen 2 parallel zur Einsetzrichtung 4 verläuft.

Die Pfanne 1 wird auf folgende Weise in den Beckenknochen des Patienten eingesetzt:

Zunächst wird ein Hohlraum zur Aufnahme der Pfanne 1 in den Beckenknochen des Patienten eingefräst, wie dies aus dem Stand der Technik bekannt ist. Anschließend wird mit einer geeigneten Bohrlehre eine Anzahl von Bohrungen zur späteren Aufnahme der Stifte 3 in den Beckenknochen des Patienten gebohrt, wobei anhand von Röntgenuntersuchungen die optimale Anordnung und Tiefe der Bohrungen bestimmbar ist.

Die Tiefe einer jeweils eingebrachten Bohrung kann mit Hilfe aus dem Stand der Technik bekannter Instrumente überprüft und sichergestellt werden.

Anschließend wir die Pfanne 1 mit Stiften 3 in der vorgesehenen Art bestückt. Dann kann die Pfanne 1 mitsamt den Stiften 3 in den Beckenknochen des Patienten eingesetzt werden. Insbesondere wenn der Durchmesser der Bohrungen im Beckenknochen des Patienten geringfügig kleiner ist als der Durchmesser der Stifte 3, ergibt sich dabei ein Druck, der die Stifte 3 in den Bohrungen 2 der Pfanne 1 zunehmend stärker verankert.

Insgesamt ist die Dauer, die zur Vorbereitung des Einsetzens und zum Einsetzen selbst der Pfanne 1 in den Beckenknochen des Patienten erforderlich ist, relativ kurz und für den Patienten dementsprechend schonend.

Bei eingesetzter Pfanne 1 in den Beckenknochen des Patienten ist ebenfalls eine Schonung für den Patienten dadurch erzielt, daß die konische Ausgestaltung der Bohrungen 2 verhindert, daß die Stifte 3 ins Innere der Pfanne 1 gelangen können, auch wenn sich die Stifte 3 im Beckenknochen des Patienten lockern sollten oder wenn sich die Pfanne 1 aufgrund starker Druckbeanspruchung ins Innere des Beckenknochens einarbeiten sollte.

In jedem Fall ist ausgeschlossen, daß die Stifte 3 ins Innere der Pfanne 1 gelangen können oder dorthin so weit vorstehen können, daß sie zu einem Abrieb an einer Innenschale führen können, die als Gleitschale im Inneren der Pfanne 1 vorgesehen sein kann. Da auf diese Weise Abriebpartikel verhindert werden, die zu lokalen Entzündungen führen können, wobei durch die Entzündungen das Knochengewebe angegriffen werden kann, wird durch die erfindungsgemäße Pfanne nicht nur beim Einsetzen selbst, sondern auch beim späteren Tragen eine Schonung für den Patienten ermöglicht.

Bei der Entnahme des Knochenimplantates werden entweder die Stifte 3 zusammen mit der Pfanne 1 aus dem Beckenknochen des Patienten herausgezogen, wenn aufgrund des Konussitzes eine feste Verbindung zwischen den Stiften 3 und der Pfanne 1 besteht. Oder die Stifte 3 verbleiben in der Knochensubstanz, wenn dort die Haltekräfte größer sind als im Bereich der Bohrungen 2. In diesem Fall können die frei in den Knochenhohlraum ragenden konischen Bereiche der Stifte 3 mit einer geeigneten Zange erfaßt und gezogen werden, nachdem die Pfanne 1 - und ggf. ein oder mehrere damit fest verbundene Stifte - entgegen der Einsetzrichtung 4 herausgezogen wurde.

## Patentansprüche

1. Knochenimplatat,
mit einer im wesentlichen kugelsegmentartig ausgebildeten Pfanne (1) zur Aufnahme des Gelenkkopfes einer Oberschenkel-Knochenprothese,
sowie mit stiftartigen Befestigungsmitteln (3) zur Verankerung der Pfanne (1) im Beckenknochen des Patienten,
wobei die Pfanne (1) Ausnehmungen (2) zur Aufnahme der Befestigungsmittel (3) aufweist, die Ausnehmungen (2) für eine achsparallele Anordnung der Befestigungsmittel (3) ausgebildet sind, und
die Ausnehmungen Anschläge für eine zum Inneren der Pfanne gerichtete Bewegung der Befestigungsmittel aufweisen,
**dadurch gekennzeichnet**, daß
das Knochenimplantat als während der Operation zusammenfügbarer Bausatz ausgestaltet ist, der einerseits die Pfanne (1) und andererseits die Befestigungsmittel (3) beinhaltet,
die Ausnehmungen (2) zum Inneren der Pfanne (1) konisch zulaufen, und
die Befestigungsmittel (3) in dem Bereich konisch ausgebildet sind, der in die Pfanne (1) einführbar ist,
wobei die Konizität der Ausnehmungen (2) und der Befestigungsmittel (3) gleich groß ist.

2. Knochenimplantat nach Anspruch 1, **dadurch gekennzeichnet**, daß die Befestigungsmittel (3) als in dem Bereich gewindelose Befestigungsmittel ausgebildet sind, welcher in den Beckenknochen des Patienten einführbar ist.

3. Knochenimplantat nach Anspruch 1 oder 2, **dadurch gekennzeichnet**, daß die Befestigungsmittel (3) unterschiedliche Längen aufweisen.

4. Knochenimplantat nach einem der vorhergehenden Ansprüche, **dadurch gekennzeichnet**, daß die Anzahl der Ausnehmungen (2) größer ist als die Anzahl der an der Pfanne (1) befestigten Befestigungsmittel (3).

5. Verfahren zur Herstellung eines Knochenimplantats,
welches eine im wesentlichen kugelsegmentartig ausgebildete Pfanne (1) zur Aufnahme des Gelenkkopfes einer Oberschenkel-Knochenprothese sowie stiftartige Befestigungsmittel (3) zur Verankerung der Pfanne in zur Aufnahme dieser Befestigungsmittel (3) bestimmten Bohrlöchern im Bekkenknochen des Patienten aufweist,
wobei die Pfanne (1) Ausnehmungen (2) zur Aufnahme der Befestigungsmittel (3) aufweist, die Ausnehmungen (2) für eine achsparallele Anordnung der Befestigungsmittel (3) ausgebildet sind, und die Ausnehmungen Anschläge für eine zum Inneren der Pfanne gerichtete Bewegung der Befestigungsmittel aufweisen,
**dadurch gekennzeichnet**, daß
die Pfanne (1) mit den Befestigungsmitteln (3) bestückt wird, indem die Befestigungsmittel (3) von außen in die Ausnehmungen (2) der Pfanne eingesetzt und dort mittels einer konischen Verbindung festgelegt werden,
wobei die konische Verbindung dadurch gewährleistet ist, daß die Ausnehmungen (2) zum Inneren der Pfanne kcnisch zulaufen und die Befestigungsmittel (3) in dem Bereich konisch ausgebildet sind, der in die Pfanne (1) einführbar ist, wobei die Konizitäten von Ausnehmungen (2) und Befestigungsmitteln (1) gleich groß sind.

## Claims

1. A bone implant,
having a cup (1) with a substantially spherical segmental configuration for receiving the head of a femoral bone prosthesis,
as well as with pin-like fixation means (3) for anchoring the cup (1) in the patient's pelvic bone, the cup (1) having recesses (2) for receiving the fixation means (3),
the recesses (2) being constructed for an axially parallel arrangement of the fixation means (3),
the recesses comprising stops for a movement of the fixation means directed to the interior of the cup,
**characterised in that**
the bone implant is configured as a kit that can be assembled during the operation, which contains firstly the cup (1) and secondly the fixation means (3),
the recesses (2) conically taper to the interior of the cup (1), and
the fixation means (3) have a conical configuration in the region which can be introduced into the cup (1),
the conical shape of the recesses (2) and of the fixation means (3) being of equal size.

2. A bone implant according to Claim 1,
**characterised in that** the fixation means (3) are constructed as fixation means without screw threads in the region which can be introduced into the patient's pelvic bone.

3. A bone implant according to Claim 1 or 2,
**characterised in that** the fixation means (3) have different lengths.

4. A bone implant according to one of the preceding Claims,
**characterised in that** the number of recesses (2) is larger than the number of fixation means (3) fixed to the cup (1).

5. A method for the manufacture of a bone implant,
which comprises a cup (1) having a substantially spherical segmental configuration for receiving the head of a femoral bone prosthesis as well as pin-like fixation means (3) for anchoring the cup in bore holes intended to receive these fixation means (3) in the patient's pelvic bone,
the cup (1) having recesses (2) for receiving the fixation means (3), the recesses (2) being configured for an axially parallel arrangement of the fixation means (3), and the recesses having stops for a movement of the fixation means directed to the interior of the cup,
**characterised in that** the cup (1) is covered with the fixation means (3) by the fixation means (3) being inserted from outside into the recesses (2) of the cup and being fixed there by means of a conical connection,
the conical connection being guaranteed in that the recesses (2) taper conically to the interior of the cup and the fixation means (3) have a conical configuration in the region which can be introduced into the cup (1), the conical shapes of recesses (2) and fixation means (1) being of the same size.

## Revendications

1. Implant osseux, comprenant
une cupule (1) ayant sensiblement la configuration d'un segment de sphère, destinée à recevoir la tête d'articulation d'une prothèse osseuse de fémur,
ainsi que des moyens de fixation (3) en forme de broche, destinés à l'ancrage de la cupule (1) dans l'os du bassin du patient,
la cupule (1) présentant des évidements (2) destinés à recevoir les moyens de fixation (3),
les évidements (2) étant conformés pour obtenir une disposition des moyens de fixation (3) parallèlement à l'axe, et
les évidements présentant des butées pour un mouvement des moyens de fixation dirigé vers l'intérieur de la cupule,
**caractérisé en ce que**
l'implant osseux présente la configuration d'un jeu d'éléments qui peut être assemblé pendant l'opération, et qui comprend, d'une part, la cupule (1) et, d'autre part, les moyens de fixation (3),
les évidements (2) se terminent avec une forme conique en direction de l'intérieur de la cupule (1) et
les moyens de fixation (3) sont de configuration conique dans la région qui peut être insérée dans la cupule (1),
la conicité des évidements (2) et celle des moyens de fixation (3) étant identiques.

2. Implant osseux selon la revendication 1, **caractérisé en ce que** les moyens de fixation (3) sont constitués par des moyens de fixation dépourvus de filetage dans la région qui peut être insérée dans l'os du bassin du patient.

3. Implant osseux selon la revendication 1 ou 2, **caractérisé en ce que** les moyens de fixation (3) possèdent des longueurs différentes.

4. Implant osseux selon une des revendications précédentes, **caractérisé en ce que** le nombre des évidements (2) est plus grand que le nombre des moyens de fixation (3) fixés dans la cupule (1).

5. Procédé de fabrication d'un l'implant osseux, qui comprend une cupule (1) sensiblement en forme de segment de sphère, destinée à recevoir la tête d'articulation d'une prothèse osseuse de fémur, ainsi que des moyens de fixation (3) en forme de broche destinés à l'ancrage de la cupule dans des perçages destinés à recevoir ces moyens de fixation (3), qui sont formés dans l'os du bassin du patient,
la cupule (1) présentant des évidements (2) destinés à recevoir les moyens de fixation (3), les évidements (2) étant configurés pour assurer une disposition des moyens de fixation (3) parallèle à l'axe et les évidements présentant des butées pour un mouvement des moyens de fixation dirigé vers l'intérieur de la cupule,
**caractérisé en ce que** la cupule (1) est équipée des moyens de fixation (3) par le fait que les moyens de fixation (3) sont mis en place dans les évidements (2) de la cupule en agissant de l'extérieur et y sont fixés au moyen d'une liaison conique,
la liaison conique étant garantie par le fait que les évidements (2) se terminent avec une forme conique en direction de l'intérieur de la cupule et que les moyens de fixation (3) sont de configuration conique dans la région qui peut être insérée dans la cupule (1), les conicités des évidements (2) et des moyens de fixation (1) étant identiques entre elles.
